# FASCICULE DE BREVET EUROPEEN

(11) **EP 0 657 213 B1**
(45) Date de publication et mention de la délivrance du brevet: **05.07.2000**
(21) Numéro de dépôt: 94203496.8
(22) Date de dépôt: 01.12.1994
(51) Int. Cl.: B01J 27/122, B01J 27/138, B01J 27/10, C07C 17/156

(54) **Composition catalytique et procédé d'oxychloration de l'éthylène utilisant une telle composition**
Katalytische Zusammensetzung und Verfahren zur Oxychlorierung von Ethylen unter Verwendung derselben
Catalytic composition and ethylene oxychlorination process using the same

(30) Priorité: 08.12.1993 BE 9301354
(43) Date de publication de la demande: 14.06.1995
(73) Titulaire: SOLVAY (Société Anonyme), 1050 Bruxelles (BE)
(72) Inventeur: Derleth, Helmut, D-3070 Nienburg (DE); Adem, Deniz, B-7133 Binche (BE); Strebelle, Michel, B-1150 Bruxelles (BE)
(74) Mandataire: Jacques, Philippe

(56) Documents cités:
- EP-A- 0 255 156
- EP-A- 0 375 202
- EP-A- 0 494 474
- DE-B- 1 262 262
- US-A- 5 004 849

## Description

La présente invention se rapporte à une composition catalytique utilisable en oxychloration et à un procédé d'oxychloration de l'éthylène utilisant une telle composition catalytique.

L'oxychloration, c'est-à-dire la chloration d'hydrocarbures par du chlorure d'hydrogène en présence d'air ou d'oxygène, constitue une réaction connue de longue date qui s'effectue habituellement en présence de catalyseurs constitués par des sels métalliques déposés sur des supports inertes tels que des alumines, des gels de silice, des oxydes mixtes ou encore des argiles ou autres supports d'origine naturelle. Industriellement, le catalyseur est utilisé le plus fréquemment en lit fluide mais peut aussi être employé en lit fixe. Comme sels métalliques, on utilise le plus souvent des halogénures tels que le chlorure de cuivre. Lorsqu'il est utilisé seul, le chlorure de cuivre présente toutefois l'inconvénient d'être relativement volatil, ce qui entraîne une chute de l'activité catalytique et du rendement de la réaction d'oxychloration, inacceptable dans les installations industrielles.

Il est bien connu d'améliorer les performances des catalyseurs d'oxychloration constitués de chlorure de cuivre sur support par ajout de chlorures de métaux alcalins, de métaux alcalino-terreux ou de terres rares (lanthanides). En particulier, des compositions catalytiques pour l'oxychloration comprenant simultanément des chlorures de cuivre, de magnésium et de métaux alcalins sur alumine ont déjà été proposées.

La demande EP-A-0255156 de SOLVAY décrit des compositions catalytiques ternaires contenant un mélange des chlorures de cuivre, de magnésium et d'un métal alcalin choisi parmi le sodium ou le lithium utilisés dans des proportions précises qui permettent d'atteindre un très bon rendement en 1,2-dichloréthane dans un procédé en lit fluide d'oxychloration de l'éthylène, en réduisant simultanément la corrosion des réacteurs en acier inoxydable, grâce, notamment à une réduction du collage et du mottage des grains de catalyseur.

La demande EP-A-0375202 envisage des compositions catalytiques ternaires à base des chlorures de cuivre, de magnésium et de potassium, contenant 30 à 90 g de cuivre, de 2 à 30 g de magnésium et de 2 à 30 g de potassium par kilo de composition catalytique, avec un rapport atomique Cu : Mg : K de 1 : 0,1-1,0 : 0,1-1,0.

Il a cependant été observé que la plupart des compositions de l'art antérieur comprenant simultanément des chlorures de cuivre, de magnésium et de métaux alcalins déposés sur alumine, provoquent, dans les réacteurs d'oxychloration de l'éthylène en lit fluidisé, le dépôt de salissures à la surface des tubes du faisceau de l'échangeur thermique disposé dans le lit fluide. Ce phénomène a été observé en particulier dans les procédés à l'oxygène, procédés dans lesquels l'oxygène est mis en oeuvre soit sous forme pure, soit sous la forme d'un mélange d'oxygène et d'azote plus riche en oxygène que l'air. Ce comportement des compositions catalytiques constitue un obstacle important à leur utilisation. En effet, il se forme peu à peu une couche de salissures de plus en plus épaisse à la surface des tubes, ce qui entraîne une dégradation progressive du transfert thermique. En outre, ce phénomène peut, à terme, induire de la corrosion. Il est dès lors indispensable de procéder à des arrêts réguliers des réacteurs pour nettoyer les tubes du faisceau de l'échangeur thermique.

Un des objectifs de la présente invention est dès lors de procurer des compositions catalytiques particulièrement performantes qui permettent d'atteindre, dans un procédé d'oxychloration de l'éthylène en lit fluide, un rendement en 1,2-dichloréthane élevé sans provoquer le dépôt de salissures à la surface des tubes du faisceau de l'échangeur thermique, spécialement dans les procédés à l'oxygène.

En conséquence, la présente invention concerne une composition catalytique comprenant des chlorures de cuivre, de magnésium et de métal alcalin déposés sur une alumine, utilisable en oxychloration, qui se caractérise en ce que le métal alcalin est du césium.

Elle concerne aussi un procédé d'oxychloration de l'éthylène en 1,2-dichloréthane qui se caractérise en ce que la réaction d'oxychloration est catalysée par une composition catalytique selon l'invention.

Généralement, la composition catalytique selon l'invention contient, exprimés sous forme métallique, de 30 à 90 g de cuivre, de 10 à 30 g de magnésium et de 0,05 à 15 g, de préférence de 0,1 à 10 g, de césium par kilo de composition catalytique.

De manière surprenante, on a maintenant observé que des compositions catalytiques contenant des chlorures de cuivre, de magnésium et de césium, dans les quantités spécifiées ne provoquent pas, à la surface des tubes du faisceau de l'échangeur thermique disposé dans le lit fluide, le dépôt de salissures observé avec les compositions de l'art antérieur, tout en permettant d'atteindre, en oxychloration de l'éthylène en 1,2-dichloréthane, une sélectivité en 1,2-dichloréthane par rapport à l'éthylène converti et un rendement en 1,2-dichloréthane par rapport au chlorure d'hydrogène mis en oeuvre similaires, voire meilleurs, à ceux obtenus avec les compositions de l'art antérieur.

Les compositions catalytiques selon l'invention contiennent au moins 30 g de cuivre par kilo de composition catalytique, de manière préférée au moins 40 g par kilo et, de manière particulièrement préférée, au moins 50 g par kilo. Elles contiennent au plus 90 g de cuivre par kilo de composition catalytique. Celles en contenant au plus 80 g par kilo apparaissent avantageuses. Celles en contenant au plus 70 g par kilo apparaissent particulièrement avantageuses.

Les compositions catalytiques selon l'invention contiennent au moins 10 g de magnésium par kilo de composition catalytique, de manière préférée au moins 12 g par kilo et, de manière particulièrement préférée, au moins 15 g par kilo. Elles contiennent au plus 30 g de magnésium par kilo de composition catalytique. Celles en contenant au plus 25 g par kilo apparaissent avantageuses. Celles en contenant au plus 20 g par kilo apparaissent particulièrement avantageuses.

Les compositions catalytiques selon l'invention contiennent préférentiellement au moins 0,1 g de césium par kilo de composition catalytique, de manière particulièrement préférée au moins 0,5 g par kilo et, de manière tout particulièrement préférée, au moins 1 g par kilo. Elles contiennent préférentiellement au plus 10 g de césium par kilo de composition catalytique. Celles en contenant au plus 9 g par kilo apparaissent avantageuses. Celles en contenant au plus 6 g par kilo apparaissent particulièrement avantageuses.

De bons résultats en oxychloration de l'éthylène ont été obtenus avec des compositions catalytiques contenant de 40 à 80 g de cuivre, de 12 à 25 g de magnésium et de 0,5 à 9 g de césium par kilo de composition catalytique.

Dans les compositions selon l'invention, le rapport atomique Mg/Cu est de préférence au moins de 0,3 et, de manière particulièrement préférée, au moins de 0,5. Avantageusement, ce rapport ne dépasse pas 1,5. Très avantageusement, il ne dépasse pas 1,0.

Le rapport atomique Cs/Cu est de préférence au moins de 0,003 et, de manière particulièrement préférée, au moins de 0,005. Avantageusement, ce rapport ne dépasse pas 0,10. Très avantageusement, il ne dépasse pas 0,08.

De très bons résultats en oxychloration de l'éthylène ont été obtenus avec des compositions présentant des rapports atomiques Cu : Mg : Cs de 1 : 0,5-1,0 : 0,005-0,05.

Dans une variante conforme à l'invention, la composition catalytique contient en outre du potassium. Dans cette variante, la composition catalytique contient de 0,05 à 15 g, de préférence de 0,1 à 10 g de potassium par kilo de composition catalytique.

Lorsque du potassium est présent dans la composition catalytique, le rapport atomique K/Cu est de préférence au moins de 0,01 et, de manière particulièrement préférée, au moins de 0,025. Avantageusement, ce rapport ne dépasse pas 0,30. Très avantageusement, il ne dépasse pas 0,25.

Les compositions selon l'invention constituées essentiellement des chlorures de cuivre, de magnésium et de césium déposés sur alumine sont toutefois préférées.

L'alumine mise en oeuvre comme support dans les compositions catalytiques de l'invention peut être de toute origine et être obtenue selon tout procédé connu; on utilise habituellement des alumines de type êta ou gamma. De bons résultats ont été obtenus avec une alumine gamma. L'alumine mise en oeuvre dans les compositions catalytiques de l'invention présente généralement un diamètre moyen des particules compris entre 10 et 200 µm et de préférence un diamètre moyen compris entre 20 et 120 µm. Sa surface spécifique, mesurée suivant la méthode B.E.T. est généralement comprise entre 50 m²/g et 250 m²/g. De bons résultats en oxychloration de l'éthylène ont été obtenus avec une alumine présentant une surface spécifique de 100 m²/g à 210 m²/g. Enfin, le volume poreux des alumines habituellement utilisées se situe entre 0,1 et 1 cm³/g. De préférence, le volume poreux est compris entre 0,2 et 0,8 cm³/g et de bons résultats en oxychloration de l'éthylène ont été obtenus avec une alumine présentant un volume poreux de 0,3 à 0,6 cm³/g.

Le mode d'obtention des compositions catalytiques selon l'invention n'est pas critique. Les chlorures métalliques peuvent être introduits dans la composition catalytique soit directement sous forme de chlorures, par exemple par imprégnation du support à l'aide d'une solution renfermant un mélange de ces sels, soit sous forme d'autres composés des métaux, tels que les oxydes, les hydroxydes, les nitrates ou tout autre composé capable d'être transformé en chlorure dans les conditions dans lesquelles sont menées les réactions d'oxychloration. La préparation des compositions catalytiques peut notamment être réalisée dans un tambour rotatif ou dans un lit fluidisé, par imprégnation de l'alumine avec une solution des chlorures métalliques, en une ou en plusieurs passes, en présence ou non d'additifs tels que des acides, par exemple l'acide chlorhydrique.

Un mode d'obtention ayant donné de bons résultats consiste à imprégner une alumine avec une solution aqueuse renfermant les quantités adéquates des chlorures de cuivre, de magnésium et de césium dans lequel on évite l'apparition d'une phase liquide non adsorbée par le solide en limitant le volume de la solution imprégnante à 70 à 100 % du volume poreux de la quantité d'alumine mise en oeuvre. L'alumine imprégnée est ensuite séchée avant d'être introduite dans le réacteur d'oxychloration proprement dit.

Les compositions catalytiques finales présentent généralement une surface spécifique B.E.T. comprise entre 25 m²/g et 200 m²/g et de préférence entre 50 et 150 m²/g. De bons résultats en oxychloration de l'éthylène ont été obtenus avec des compositions catalytiques présentant une surface spécifique B.E.T. de 80 à 140 m²/g.

Les compositions catalytiques selon l'invention sont particulièrement avantageuses dans un procédé d'oxychloration dans lequel le catalyseur est sous forme de lit fluidisé. Elles peuvent néanmoins aussi être mises en oeuvre dans un procédé d'oxychloration réalisé avec un catalyseur disposé en lit fixe, moyennant mise en forme appropriée des particules de catalyseur, par exemple sous forme de granules de quelques mm de diamètre. Les compositions catalytiques selon l'invention conviennent pour les procédés d'oxychloration à l'air ou à l'oxygène. Elles sont particulièrement bien adaptées au procédé à l'oxygène, utilisant de l'oxygène pur ou un mélange oxygène/azote plus riche en oxygène que l'air.

Les compositions catalytiques selon l'invention conviennent tout particulièrement pour un procédé d'oxychloration à l'oxygène de l'éthylène en 1,2-dichloréthane dans lequel le catalyseur est sous forme de lit fluidisé. Un tel procédé recourant à une composition catalytique selon l'invention est particulièrement préféré.

Lorsque l'on opère avec un catalyseur disposé en lit fluidisé, la température à laquelle s'effectue la réaction d'oxychloration se situe habituellement entre 200 et 300 °C. De préférence, cette température est comprise entre 220 et 280 °C. Enfin, des bons résultats ont été obtenus à une température située aux environs de 230 - 270 °C.

La pression à laquelle est effectuée la réaction d'oxychloration n'est pas critique en elle-même. Habituellement, on opère avec des pressions comprises entre 0,1 et 1 MPa et de préférence avec des pressions comprises entre 0,1 et 0,8 MPa. La vitesse de fluidisation des compositions catalytiques n'est pas critique en elle-même et dépend essentiellement de la granulométrie du catalyseur et des dimensions de l'appareillage. Généralement, on opère avec des vitesses comprises entre 5 et 100 cm/s. Enfin, le rapport des réactifs mis en oeuvre est le même que celui généralement utilisé dans les procédés antérieurs. D'habitude, on opère avec un léger excès d'éthylène par rapport à la quantité stoechiométrique nécessaire pour réagir avec l'HCl mis en oeuvre. Toutefois, les compositions catalytiques de l'invention permettent indifféremment de travailler avec des excès importants d'éthylène ou au voisinage de la stoechiométrie, voire même en excès d'HCl.

L'invention se trouve plus amplement illustrée par les exemples suivants. Les exemples annotés (c) se rapportent à des exemples donnés à titre comparatif.

### Exemples 1 à 6

Un catalyseur selon l'invention a été préparé au départ d'une alumine gamma présentant une surface spécifique de 186 m²/g, un volume poreux de 0,38 cm³/g, un poids spécifique (mesuré par écoulement libre) de 0,75 kg/dm³ et un diamètre moyen des particules de 50 µm. A environ 800 g de cette alumine a été ajoutée une solution aqueuse d'imprégnation comprenant à l'état dissous du CuCl₂.2H₂O, du MgCl₂.6H₂O et du CsCl en quantités adéquates pour obtenir, après séchage à 150 °C, environ 1 kg de catalyseur présentant, calculée sous forme métallique par rapport au poids total du catalyseur, une teneur en cuivre de 60 g/kg, une teneur en magnésium de 17 g/kg et une teneur en césium de 1 g/kg. Exprimée en rapport atomique, la proportion entre les différents métaux Cu : Mg : Cs est de 1 : 0,74 : 0,008.

Un deuxième catalyseur selon l'invention et quatre autres catalyseurs, constitués uniquement de chlorure de cuivre et de chlorure de césium sur alumine ou de chlorure de cuivre et de chlorure de magnésium sur alumine ont été préparés selon la même procédure. Les teneurs en métaux dans ces différents catalyseurs sont rassemblées au tableau I.

Ces 6 catalyseurs ont été testés en oxychloration de l'éthylène dans un réacteur micro-pilote en lit fluide, contenant 280 cm³ de catalyseur. Le catalyseur est fluidisé au moyen des gaz réactifs introduits par le bas au travers d'un filtre métallique fritté. Les conditions opératoires dans lesquelles les tests ont été réalisés sont les suivantes :
- rapport 2C₂H₄/HCl = 1,04 mol/mol
- rapport 4O₂/HCl variable, de manière à maintenir 1 % volume d'O₂ dans les gaz de queue.
- vitesse des gaz : 12,5 cm/s (par rapport au réacteur vide à pression et température d'essai)
- température : 255 °C
- pression : 0,6 MPa
- temps de séjour : 5 s.

Les produits de réaction quittant le réacteur ont été détendus jusqu'à la pression atmosphérique par une vanne de régulation de pression du réacteur et refroidis dans un piège maintenu à - 20 °C. Les gaz non condensés ont été lavés dans un scrubber à eau avant de balayer une ampoule de prélèvement. Le bilan des produits formés a été effectué au départ d'analyses chromatographiques des produits liquide et gazeux recueillis et du titrage de l'acidité de la solution aqueuse recueillie au pied du scrubber. Le rendement en 1,2-dichloréthane (rapport molaire entre le DCEa formé et l'HCl mis en oeuvre) et la sélectivité en DCEa (rapport molaire entre le DCEa formé et l'éthylène converti) sont présentés au tableau I.

Le dépôt de salissures provoqué par les différents catalyseurs a été mesuré dans un réacteur micro-pilote similaire au réacteur décrit ci-avant mais muni en outre d'un tube en forme de doigt de gant, plongeant verticalement dans le lit fluide. Ce tube en doigt de gant comporte une double paroi dans laquelle circule une huile maintenue à une température inférieure à la température à laquelle est menée la réaction. Le dépôt de salissures est déterminé visuellement par examen de la surface externe de ce tube en doigt de gant après 20 heures de fonctionnement du réacteur dans les conditions suivantes :
- rapport 2C₂H₄/HCl = 1,07 mol/mol
- rapport 4O₂/HCl = 1,12
- vitesse des gaz : 2,5 cm/s
- température dans le lit fluide : 270 °C
- température à la surface externe du tube en doigt de gant : 180 °C.
- pression : 0,7 MPa
- temps de séjour : 12 s.

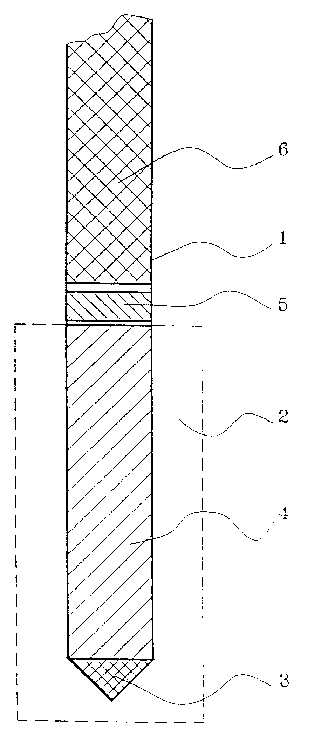

Dans ces conditions, les résultats obtenus reflètent le comportement des catalyseurs observé après quelques mois de fonctionnement dans un réacteur industriel. Une cote chiffrée est attribuée aux catalyseurs, en fonction de l'aspect des salissures et de l'endroit auquel elles apparaissent à la surface externe du tube en doigt de gant. A la figure unique, on a schématisé le tube en doigt de gant (1) plongeant dans le lit fluide (2). Le tube comporte 4 zones distinctes : une pointe conique (3), une surface cylindrique (4) plongeant dans le lit fluide (2), une interface (5), située juste au-dessus du lit fluide et une surface cylindrique (6), située hors du lit fluide, au-dessus de l'interface (5). La présence d'un voile, c'est-à-dire d'une fine pellicule adhérente qui ne comprend pas de particules de catalyseur, à la pointe (3) ou à la surface (4) plongeant dans le lit fluide vaut 1 point. La présence d'une croûte, c'est-à-dire d'un dépôt plus épais comprenant des particules de catalyseur adhérant à la surface du tube, vaut 2 points à la pointe (3) et à la surface (4) et 1 point à l'interface (5). Sur la zone de la surface (6) située hors du lit, seule la présence d'agrégats de particules de catalyseurs a parfois été observée et est comptée pour 1 point. La présence sur n'importe quelle zone de la surface du tube de particules de catalyseur non adhérentes n'est pas prise en compte. Enfin, une mauvaise fluidisation du catalyseur entraînant sa prise en masse compte pour 6 points. Une cote de 0 sera donc attribuée à un catalyseur ne provoquant aucun dépôt de salissures lors du test, alors qu'un catalyseur provoquant une apparition importante de salissures, mise en évidence, par exemple, par la présence de croûtes à la pointe (3) (2 points), à la surface (4) (2 points) et à l'interface (5) (1 point) se verra attribuer une cote de 5.

Les résultats obtenus sont rassemblés au tableau I.

Il ressort clairement de la comparaison entre les exemples 1 et 2 selon l'invention et les exemples 3(c) à 6(c) de comparaison que la présence simultanée de chlorure de magnésium et de chlorure de césium dans la composition catalytique permet d'obtenir de bonnes performances chimiques (rendement et sélectivité) sans provoquer le dépôt de salissures, ce qui n'est pas le cas lorsque seul un de ces deux chlorures est présent dans la composition catalytique, même à une teneur plus importante.

### Exemples 7 à 15

Les catalyseurs mis en oeuvre dans les exemples 7 à 15 ont été préparés de la même manière que les catalyseurs des exemples 1 à 6, au départ de la même alumine imprégnée par une solution aqueuse renfermant CuCl₂.2H₂O, MgCl₂.6H₂O et CsCl, KCl, LiCl ou NaCl en quantités et proportions adéquates.

Les différents catalyseurs ont été testés en oxychloration de l'éthylène dans le réacteur micro-pilote en lit fluide utilisé aux exemples 1 à 6, dans les conditions opératoires suivantes :
- volume de catalyseur : 225 cm³
- rapport 2C₂H₄/HCl : 1,07 mol/mol
- rapport 4O₂/HCl : 1,35 mol/mol
- vitesse des gaz : 10 cm/s (par rapport au réacteur vide à pression et température d'essai)
- température : 260 °C
- pression : 0,6 MPa
- temps de séjour : 5 s.

Le dépôt de salissures a été mesuré selon la même procédure que celle utilisée dans les tests des exemples 1 à 6.

Le tableau II ci-après reprend les compositions des différents catalyseurs testés, les résultats obtenus en oxychloration de l'éthylène ainsi que les mesures de dépôt de salissures.

Les compositions catalytiques des exemples 10 à 15 de comparaison, donnent un bon rendement en 1,2-dichloréthane par rapport à l'HCl et une bonne sélectivité de l'éthylène en 1,2-dichloréthane mais provoquent le dépôt de salissures à la surface du tube en doigt de gant. Par contre, les exemples 7 à 9 démontrent que les compositions selon l'invention ne provoquent aucun dépôt de salissures, tout en procurant une sélectivitè et un rendement en 1,2-dichloréthane très élevés.

### Exemple 16

Un catalyseur contenant du cuivre, du magnésium, du césium et du potassium a été préparé de manière similaire aux catalyseurs mis en oeuvre dans les essais 1 à 15. Ce catalyseur contenait 59 g de cuivre, 17 g de magnésium, 1,7 g de césium et 3 g de potassium par kilo. Il a été testé dans les mêmes conditions que celles des exemples 1 à 6.

Dans ces conditions, cette composition ne provoque aucun dépôt de salissures (cote : 0) et procure une sélectivité en DCEa par rapport à l'éthylène converti remarquablement élevée (98,4 % mol), tout en procurant un rendement en 1,2-dichloréthane par rapport à l'HCl acceptable (97 % mol).

**Tableau I**

| N° Ex. | Composition | | | | | | Rendement en DCEa par rapport à HCl (%mol) | Sélectivité en DCEa par rapport à l'éthylène converti (%mol) | Dépôt de salissures (voir texte) |
|---|---|---|---|---|---|---|---|---|---|
| | teneur poids (g/kg) | | | proportions atomiques | | | | | |
| | Cu | Mg | Cs | Cu | Mg | Cs | | | |
| 1 | 60 | 17 | 1 | 1 | 0,74 | 0,008 | 98,3 | 96,7 | 0 |
| 2 | 62 | 18 | 2,6 | 1 | 0,76 | 0,020 | 98,6 | 97,7 | 0 |
| 3(c) | 60 | 17 | - | 1 | 0,74 | - | 98,2 | 96,8 | 3 |
| 4(c) | 60 | 24 | - | 1 | 1,05 | - | 96,5 | 98,1 | 3 |
| 5(c) | 60 | - | 1 | 1 | - | 0,008 | 97,7 | 94,2 | 10 |
| 6(c) | 60 | - | 3 | 1 | - | 0,024 | 97,7 | 94,6 | 10 |

**Tableau II**

| N° Ex. | Composition | | | | | | Rendement en DCEa par rapport à HCl (%mol) | Sélectivité en DCEa par rapport à l'éthylène converti (%mol) | Dépôt de salissures (voir texte) |
|---|---|---|---|---|---|---|---|---|---|
| | teneur poids (g/kg) | | | proportions atomiques | | | | | |
| | Cu | Mg | Alc | Cu | Mg | Alc | | | |
| 7 | 60 | 17 | 1Cs | 1 | 0,74 | 0,008Cs | 98,3 | 96,2 | 0 |
| 8 | 60 | 17 | 4,6Cs | 1 | 0,74 | 0,04Cs | 97,8 | 97,3 | 0 |
| 9 | 60 | 17 | 8,7Cs | 1 | 0,74 | 0,07Cs | 97,2 | 97,6 | 0 |
| 10(c) | 60 | 17 | 2,1Li | 1 | 0,74 | 0,33Li | 97,5 | 94,9 | 3 |
| 11(c) | 56 | 17 | 3,1Li | 1 | 0,79 | 0,51Li | 98,2 | 96,7 | 3 |
| 12(c) | 60 | 18 | 2,1Na | 1 | 0,80 | 0,10Na | 97,9 | 94,4 | 1 |
| 13(c) | 60 | 17 | 4,4Na | 1 | 0,74 | 0,21Na | 98,2 | 96,0 | 4 |
| 14(c) | 58 | 17 | 11K | 1 | 0,77 | 0,31K | 98,2 | 96,8 | 1 |
| 15(c) | 60 | 17 | 17K | 1 | 0,74 | 0,47K | 97,9 | 97,2 | 1 |

## Revendications

1. Composition catalytique comprenant des chlorures de cuivre, de magnésium et de métal alcalin déposés sur une alumine, caractérisée en ce que le métal alcalin est du césium ou du césium et du potassium.

2. Composition catalytique selon la revendication 1, contenant de 30 à 90 g de cuivre, de 10 à 30 g de magnésium et de 0,1 à 10 g de césium, exprimés sous forme métallique, par kilo de composition catalytique.

3. Composition catalytique selon la revendication 1 ou 2, contenant de 40 à 80 g de cuivre, de 12 à 25 g de magnésium et de 0,5 à 9 g de césium, exprimés sous forme métallique, par kilo de composition catalytique.

4. Composition catalytique selon l'une quelconque des revendications 1 à 3, dans laquelle le rapport atomique Cs/Cu est de 0,003 à 0,10.

5. Composition catalytique selon l'une quelconque des revendications 1 à 4, dans laquelle les rapports atomiques Cu : Mg : Cs sont de 1 : 0,5-1,0 : 0,005-0,05.

6. Composition catalytique selon l'une quelconque des revendications 1 à 5, contenant du potassium.

7. Composition catalytique selon la revendication 6, dans laquelle le rapport atomique K/Cu est de 0,01 à 0,30.

8. Composition catalytique selon l'une quelconque des revendications 1 à 7, dans laquelle l'alumine présente une surface spécifique, mesurée suivant la méthode B.E.T. comprise entre 50 m²/g et 250 m²/g.

9. Procédé d'oxychloration de l'éthylène en 1,2-dichloréthane par réaction avec du chlorure d'hydrogène en présence d'air ou d'oxygène, caractérisé en ce que la réaction d'oxychloration est catalysée par une composition catalytique selon l'une quelconque des revendications 1 à 8.

10. Procédé d'oxychloration selon la revendication 9, dans lequel la composition catalytique est sous forme de lit fluidisé.

11. Procédé d'oxychloration selon la revendication 9, dans lequel l'oxygène est mis en oeuvre est soit sous forme pure, soit sous la forme d'un mélange d'oxygène et d'azote plus riche en oxygène que l'air.

## Claims

1. Catalytic composition comprising copper chloride, magnesium chloride and an alkali metal chloride deposited on an alumina, characterized in that the alkali metal is caesium or caesium and potassium.

2. Catalytic composition according to Claim 1, containing from 30 to 90 g of copper, from 10 to 30 g of magnesium and from 0.1 to 10 g of caesium, expressed as metal, per kilo of catalytic composition.

3. Catalytic composition according to Claim 1 or 2, containing from 40 to 80 g of copper, from 12 to 25 g of magnesium and from 0.5 to 9 g of caesium, expressed as metal, per kilo of catalytic composition.

4. Catalytic composition according to any one of Claims 1 to 3, in which the Cs/Cu atomic ratio is from 0.003 to 0.10.

5. Catalytic composition according to any one of Claims 1 to 4, in which the Cu : Mg : Cs atomic ratios are 1 : 0.5-1.0 : 0.005-0.05.

6. Catalytic composition according to any one of Claims 1 to 5, containing potassium.

7. Catalytic composition according to Claim 6, in which the K/Cu atomic ratio is from 0.01 to 0.30.

8. Catalytic omposition according to any one of Claims 1 to 7, in which the alumina has a specific surface, measured according to the B.E.T. method, of between 50 m²/g and 250 m²/g.

9. Process for the oxychlorination of ethylene into 1,2-dichloroethane by reaction with hydrogen chloride in the presence of air or oxygen, characterized in that the oxychlorination reaction is catalysed by a catalytic composition according to any one of Claims 1 to 8.

10. Oxychlorination process according to Claim 9, in which the catalytic composition is in the form of a fluidized bed.

11. Oxychlorination process according to Claim 9, in which the oxygen used is either in pure form or in the form of an oxygen and nitrogen mixture which is richer in oxygen than air.

## Patentansprüche

1. Katalytische Zusammensetzung, die auf einem Aluminiumoxid abgeschiedene Chloride von Kupfer, Magnesium und Alkalimetall umfasst, dadurch gekennzeichnet, dass das Alkalimetall Cäsium oder Cäsium und Kalium ist.

2. Katalytische Zusammensetzung gemäß Anspruch 1, die 30 bis 90 g Kupfer, 10 bis 30 g Magnesium und 0,1 bis 10 g Cäsium, ausgedrückt in metallischer Form, pro Kilo katalytischer Zusammensetzung enthält.

3. Katalytische Zusammensetzung gemäß Anspruch 1 oder 2, die 40 bis 80 g Kupfer, 12 bis 25 g Magnesium und 0,5 bis 9 g Cäsium, ausgedrückt in metallischer Form, pro Kilo katalytischer Zusammensetzung enthält.

4. Katalytische Zusammensetzung gemäß einem der Ansprüche 1 bis 3, bei der das Atomverhältnis Cs/Cu 0,003 bis 0,10 beträgt.

5. Katalytische Zusammensetzung gemäß einem der Ansprüche 1 bis 4, bei der die Atomverhältnisse Cu : Mg : Cs 1 : 0,5-1,0 : 0,005-0,05 betragen.

6. Katalytische Zusammensetzung gemäß einem der Ansprüche 1 bis 5, die Kalium enthält.

7. Katalytische Zusammensetzung gemäß Anspruch 6, bei der das Atomverhältnis K/Cu 0,01 bis 0,30 beträgt.

8. Katalytische Zusammensetzung gemäß einem der Ansprüche 1 bis 7, bei der das Aluminiumoxid eine nach dem BET-Verfahren gemessene spezifische Oberfläche zwischen 50 m²/g und 250 m²/g aufweist.

9. Verfahren zur Oxychlorierung von Ethylen zu 1,2-Dichlorethan durch Reaktion mit Chlorwasserstoff in Gegenwart von Luft oder Sauerstoff, dadurch gekennzeichnet, dass die Oxychlorierungsreaktion durch eine katalytische Zusammensetzung gemäß einem der Ansprüche 1 bis 8 katalysiert wird.

10. Verfahren zur Oxychlorierung gemäß Anspruch 9, bei dem die katalytische Zusammensetzung in Form eines Fließbetts vorliegt.

11. Verfahren zur Oxychlorierung gemäß Anspruch 9, bei dem der Sauerstoff entweder in reiner Form oder in Form eines Gemisches von Sauerstoff und Stickstoff, das sauerstoffreicher als die Luft ist, verwendet wird.
